# EUROPEAN PATENT APPLICATION

(11) **EP 4 438 029 A1**
(43) Date of publication of application: **02.10.2024**
(21) Application number: 24164148.9
(22) Date of filing: 18.03.2024
(51) Int. Cl.: A61K 8/02, A61Q 1/02, A61Q 1/10

(54) **EYE COLORANT AND METHOD FOR MANUFACTURING A PATIENT INDIVIDUAL EYE COLORANT**

(30) Priority: 23.03.2023 CH 3232023
(71) Applicant: Frank Ziemer Holding AG, 2562 Port (CH)
(72) Inventor: MARASHI, Mohamad Omar, Aleppo (SY)
(74) Representative: Rentsch Partner AG

(57) **Abstract**

The present disclosure relates to an eye colorant (100) for coloring a portion of a cornea of an eye of a patient, the eye colorant comprises composite material particles (110) with coloring pigments (112) of at least one color and, a matrix material (114), which at least partially embeds the coloring pigments (112), thereby forming the composite material particles (110) and to a method for manufacturing a patient individual eye colorant (100) for coloring a portion of a cornea of an eye of a patient.

## Description

### FIELD OF THE DISCLOSURE

The present disclosure relates to an eye colorant for coloring a portion of a cornea of an eye of a patient and to a method of manufacturing a patient individual eye colorant for coloring a portion of a cornea of an eye of a patient. In particular, the present disclosure relates to an eye colorant for coloring a portion of a cornea of an eye of a patient, wherein the eye colorant comprises composite material particles, and to a method of manufacturing a patient individual eye colorant for coloring a portion of a cornea of an eye of a patient, wherein the composition of the different components of the eye colorant is selected in dependence of the iris structure and / or iris color distribution of the eye of patient.

### BACKGROUND OF THE DISCLOSURE

Each person has an individual iris color distribution and iris color structure. The individual color and structure of the iris is determined by an individual composition of pigments across the iris of the eye. The eye color distribution of humans varies normally from dark brown to light blue. Different pigment concentrations on different areas of the iris may further determine the individual appearance of the iris. For example, a light blue center of the iris may be surrounded by a darker ring on the peripheral part of the iris.

Different diseases or defects of the iris may change the color of the iris or may affect the iris color distribution or the appearance of the iris. Further, it is possible that congenital diseases or defects affect the properties and geometrical extensions of the iris of the eye, such that the iris of the patient deviates from a normal ring shape. Such congenital diseases are for example albinism, coloboma or aniridia, further congenital diseases are also conceivable. In order to at least improve or affect the visual appearance of such an affected iris, it is possible to perform a so-called keratopigmentation. Keratopigmentation describes different kind of chirurgical methods to change the appearance of the iris. For example, it is possible to place a colored implant on the iris or to depigment the iris with a laser beam. Both chirurgical methods are dangerous for the iris and in particular, the second one is limited with respect to only brightening the already existing iris.

Another possibility to amend the visual appearance of the iris is to place in the cornea of the respective eye, thus in front of the iris with respect to the visual axis of the eye, ink, such that the ink covers partially or entirely the natural color of the iris. Such a method is conventionally performed via a controlled chirurgical intervention. An ophthalmological laser device, for example a femtosecond laser, cuts a ring circular intrastromal pocket in the cornea of the eye of the patient and the desired ink is inserted into the pocket of the cornea. With such a chirurgical operation, it is possible to change partially or entirely the visual appearance of the iris color of the patient. This ink, which has the desired color, is inserted in the intrastromal pocket. The ink stays in its liquid form in the intrastromal pocket in the cornea of the eye of the patient. The conventionally used ink may bleach in the sun or may change or lose its brightness and color over time. Further, it is conceivable that the ink penetrates over time into additional areas of the cornea of the eye, which may lead to an undesired visual appearance of the treated eye.

### SUMMARY OF THE DISCLOSURE

It is an object of the present disclosure to provide an eye colorant for coloring a portion of a cornea of an eye of a patient and to provide a method for manufacturing a patient individual eye colorant. In particular, it is an object of the present disclosure to provide an eye colorant for coloring a portion of a cornea of an eye of a patient and to provide a method for manufacturing a patient individual eye colorant, which do not have at least some of the disadvantages of the prior art.

According to the present disclosure, these objects are addressed by the features of the independent claims. In addition, further advantageous embodiments follow from the dependent claims and the description.

According to the present disclosure, an eye colorant for coloring a portion of a cornea of an eye of a patient is specified. The eye colorant comprises composite material particles. The composite material particles comprise coloring pigments of at least one color and a matrix material, which at least partially embeds the coloring pigments, thereby forming the composite material particles.

The composite material particles comprise according to the present disclosure the coloring pigments and the matrix material. The coloring pigments, which define at least partially the color of the eye colorant, are embedded in the matrix material, which is preferably a solid material. The matrix material, which is for example, at least partially transparent, surrounds at least partially, preferably entirely, the coloring pigments. In case the matrix material is fully transparent, the color of the eye colorant is only determined by the coloring pigments. The combination of the coloring pigments and the matrix material in the composite material particles enables to create advantageously realistic and imitation of eye color using such eye colorant. Further, the eye colorant according to the present disclosure is advantageously stable and long lasting. Different composite material particles each comprising different coloring pigments may be combined to create the desired color and structure of the eye colorant for the eye of the patient. The eye colorant as specified is, for example, injected into the cornea of a patient at a predefined portion of the cornea of the eye. The portion is, for example, ring shaped or partially ring shaped and covers for example, the desired defected portion of the iris of the eye of the patient. The eye colorant of the present disclosure enables advantageously to cover the defect portion of the iris of the eye or to cover the entire iris of the eye. It is also conceivable that the eye colorant according to the present disclosure is not inserted directly into the cornea, but is inserted into a separate cornea sample implant. For example, the cornea sample implant, which corresponds to the desired portion to be covered of the iris, is colored by inserting the eye colorant. The colored cornea sample implant may be inserted into the cornea of the eye of the patient, for amending the visual appearance of the iris of the eye. The cornea sample implant is for example extracted from a donor eye, from the eye of the patient to be treated or from the other eye of the patient. The cornea sample implant, providing the pocket for the eye colorant, may further be manufactured from a plastic material.

In an embodiment, the eye colorant further comprises a carrier liquid, in which the composite material particles are distributed. The composite material particles are arranged and distributed in the carrier liquid, which simplifies the handling of the eye colorant. The eye colorant may be injected or inserted in liquid form into the desired position of the eye of the patient, which improves handling of the eye colorant. The carrier liquid is for example configured to evaporate or solidify after inserting, which advantageously reduces the risk that the composite material particles move around after inserting. In another embodiment, the carrier liquid is for example configured to carry the composite material particles throughout the lifetime of the eye colorant, evaporating of the carrier liquid is not foreseen. In this embodiment, the eye colorant and in particular the composite material particles may be removed, for example flashed out, from the cornea of the patient in an advantageously simple fashion.

In an embodiment, the carrier liquid comprises a balanced salt solution, a buffered saline solution and / or sterile intraocular irrigating solution, preferably balanced salt solution enriched with bicarbonate, dextrose, and/or glutathione. In an embodiment, the carrier liquid may be BSSO or BSS^{®} PLUS from Alcon Inc. In an embodiment, the carrier liquid is at least partially a sodium hyaluronate solution.

In an embodiment, the eye colorant further comprising coloring pigments, which are distributed in the carrier liquid and / or additional particles, which are distributed in the carrier liquid. According to this embodiment, coloring pigments of, for example, at least one color are additionally distributed in the carrier liquid. Further, additional particles of, for example, at least one kind, are distributed in the carrier liquid. In other words, the carrier liquid does not only comprise the composite material particles but also the coloring pigments and / or the additional particles, in particular which have no matrix material at least partially surrounding or embedding them. The additional particles have preferably a different material composition with respect to the coloring pigments and the matrix material This embodiment increases the possible color variations and structure variations of the eye colorant such that the desired color and structure of the eye colorant is advantageously creatable.

In an embodiment, the composite material particles further comprise additional particles, which are also at least partially embedded within the matrix material. In this embodiment, the composite material particles comprise the coloring pigments, the additional particles and the matrix material. The additional particles are also at least partially embedded within the matrix material. The additional particles within the composite material particles are, for example, at least partially the same as the additional particles within the carrier liquid. The additional particles within the composite material particles further increase the variety of possible colors and color structures creatable with the eye colorant.

In an embodiment, at least some of the additional particles have a reflective surface and / or wherein at least some of the additional particles have an absorbing surface. A reflective surface is a surface, which at least partially, preferably entirely, reflects incident light. Such a reflective / shiny surface advantageously helps to recreate the desired eye color and / or eye color distribution (natural iris appearance) by the eye colorant comprising such additional particles. Further, an absorbing surface is a surface, which at least partially, preferably entirely, absorbs incident light. The additional particles with the reflective and / or absorbing surface advantageously help to recreate the appearance of the individual, natural iris appearance of the eye of the patient. The reflective surface / the absorbing surface prevent or at least reduce that the artificial iris is illuminated from behind.

In an embodiment, the additional particles, comprising the reflective surface, are made of titanium dioxide. Titanium dioxide has a refractive index that is significantly higher than that of most organic substances used to bind colors. This means that the additional particles comprising titanium dioxide scatter light effectively, resulting in a well-covered white color. Here, the optimum size of the pigments is in the range of 200 nm to 300 nm. Pigments in the range of below 100 nm should be avoided due to potentially toxic effects. Additionally, the titanium dioxide particles advantageously prevent that the artificial iris is being illuminated from behind. Titanium dioxide is additionally biocompatible and is therefore advantageously usable for the reflective additional particles to create the desired shininess of the appearance of the iris of the patient. In a further embodiment, the amount or the ratio of the additional particles may be dependent on properties of the coloring pigments, in particular of the color/color distribution of the coloring pigments.

In a further embodiment, the additional particles comprising the reflective surface are made of aluminum oxide and / or silicon oxide.

In an embodiment, the additional particles comprising the absorbing surface are made of a carbon-based material. Carbon based materials are or comprise for example graphite, soot, carbon nano tubes, carbon fillers or silicon carbide. The carbon-based materials provide the desired light absorbing surface properties to create an advantageously simple and realistic fashion of the darker portions of the iris appearance.

In an embodiment, the composite material particles are coated, wherein the coating is preferably made of a biocompatible material and / or a medically or pharmaceutically active material. The coating is for example applied on the composite material particles after its formation, such that the matrix material and the coloring pigments are entirely coated. The coating prevents that the matrix material and the coloring pigments and / or the additional particles directly contact cornea tissue of the eye when inserted into the cornea of the eye. According to this embodiment, the matrix material and the coloring pigments (and additional particles) do not need to be biocompatible, because they do not contact the cornea tissue directly. This increases the useable material range for the different components of the composite material particles. The coating itself is preferably made of biocompatible material, in particular of biocompatible material for inserting into the cornea of the eye. Additionally or alternatively, the coating may be made of or comprises medically or pharmaceutically active material. The medically or pharmaceutically active material may have an anti-inflammatory effect on the cornea of the eye, which may reduce the risk of an infection of the cornea after inserting the eye colorant. The medically active material may further provide additional medical effects for the cornea of the eye after being inserted. The medical active material may comprise pharmaceutically active additives.

In an embodiment, the coating material comprises biocompatible polymers such as PMMA or silicone.

In an embodiment, the coating is applied on the composite material particles using a plastic coating process, for example a dip coating process. In another embodiment, the coating is applied on the composite material particles using a powder coating process, in particular for covering the composite material particles in a biocompatible coating.

In an embodiment, the matrix material is made of a biocompatible material and / or a transparent material and / or a medically or pharmaceutically active material. In case the composite material particles do not comprise the coating as described above or in case the coating does not cover the composite material particles entirely, it is advantageously that the matrix material itself is also biocompatible or is medically or pharmaceutically active, such that also the matrix material itself also provides the advantages as described above. The matrix material may further be partially or entirely transparent, such that it does not, or it does only slightly influence the visual appearance of the eye colorant.

In an embodiment, the composite material particles, the coloring pigments and / or the additional particles are sterilized. The particles are preferably sterilized using heat and / or gassing by means of EO (Ethylene Oxide). Sterilized particles are advantageously accepted tolerated within the cornea of the eye and reduce the risk of infections.

In a further embodiment, the carrier liquid is made of a biocompatible material and / or a transparent material and / or a medically active material. According to this embodiment, the carrier liquid may additionally or alternatively provide the advantages as described above with respect to the biocompatibility, transparency and / or medical or pharmaceutical activity.

In an embodiment, the matrix material is made of a thermoplastic material, preferably made of a polymethyl methacrylate material, or wherein the matrix material is made of a synthetic polymer material, preferably of a polyorganosiloxane material. A combination of the thermoplastic material and the synthetic polymer material is also conceivable. The thermoplastic material, in particular a polymethyl methacrylate material (PMMA) or the synthetic polymer material, in particular the polyorganosiloxane material, provide the desired properties for the matrix material, in particular the advantageous biocompatibility.

In an embodiment, the composite material particles have an average equivalent diameter of below 25 micrometer, preferably below 10 micrometer, more preferably below 5 micrometer. In an embodiment, the composite material particles have an average equivalent diameter of above 1 micrometer, preferably above 2 micrometer, more preferably above 4 micrometer. In case the composite material particles are too small, they might move within the cornea towards undesired outer or inner portions of the cornea. In case the composite material particles are too large, they might be perceived as a disturbing element in the cornea of the eye. The composite material particles having the average equivalent diameter as described above provide the desired properties. The equivalent diameter may be the equivalent spherical diameter. The average equivalent diameter is the average diameter of a number of particles of a sample of the composite material particles. In an embodiment, a mesh size of 1000 or finer is used for filtering and / or determining of the desired composite material particle size.

In an embodiment, the coloring pigments have a plurality of colors. According to this embodiment, the coloring pigments comprise a plurality of colors. In other words, different coloring pigments within the composite material particles have a different color, such that a plurality of colors are visible in the composite material particles. For example, one single composite material particle may comprise a plurality of coloring pigments of different colors, or a plurality of the composite material particles may comprise a plurality of coloring pigments of different colors, wherein each of the composite material particles comprises only coloring pigments of one single color. The plurality of colors may be created by one single kind of composite material particles having a plurality of different coloring pigments or by the multiple different kinds of composite material particles.

In an embodiment, the composition of the coloring pigments is configured to be selected in dependence of a current iris structure and / or a current iris color distribution of the eye of the patient. In a further embodiment, the composition of the coloring pigments is configured to be selected in dependence of a target iris structure and / or a target iris color distribution selected by the patient. In other words, the color distribution and / or the color structure of the eye colorant to be used is selected by the patient or by a professional in dependence of the current iris, to recreate the current appearance of the iris of the patient or in dependence of a target iris, to create a target appearance of the iris of the patient. Recreation is for example necessary in case the iris of the patient has a congenital disease or was harmed in an accident. The creation of the target iris may not be medically necessary, but may be the desire of the patient, for example to brighten the appearance of the iris.

In an embodiment, the composition of the additional particles is configured to be selected in dependence of a current iris structure and / or a current iris color distribution of the eye of the patient. In a further embodiment, the composition of the additional particles is configured to be selected in dependence a target iris structure and / or a target iris color distribution selected by the patient. In this embodiment, also the additional particles of the eye colorant are selected accordingly.

In an embodiment, the surface of the composite material particles has a hydrophobic property. Hydrophobic is a property of a substance that repels water. In other words, the surface of the composite material lacks an affinity for water, and tends to repel or not to absorb water. According to this embodiment, a wettability of the eye colorant, in particular of the composite material particles, is adjustable.

In a further embodiment, the eye colorant, in particular the carrier liquid, may comprise a curing agent or a hardener, which is configured to cure the eye colorant, preferably after injection into the cornea of the eye of the patient. The curing agent enables that the eye colorant is cured after injection. A cured eye colorant advantageously keeps its position within the cornea and is additionally removable in a simple manner.

According to a further aspect, a cornea implant comprising the eye colorant as described above or hereinafter is specified. For example, the eye colorant is injected or inserted in the cornea implant, which is in a later stage inserted in a pocket of the cornea of the eye of the patient. The cornea implant may comprise a pocket configured to accommodate the eye colorant.

According to a further aspect, a method for manufacturing a patient individual eye colorant for coloring a portion of a cornea of an eye of a patient is specified, the method comprises different steps. In a first step, at least a plurality of the different components of an eye colorant as described above and hereinafter are provided. The different components may comprise a variety of different composite material particles, coloring pigments and matrix material, carrier liquid and / or additional particles.

In a next step, a current iris structure and / or a current iris color distribution of the eye of the patient or, a target iris structure and / or a target iris color distribution is determined. This is for example performed using an analytical device, which is configured to determine the current iris structure and / or the current iris color distribution, from, for example, an image of the current iris of the patient. The target iris is for example selected by the patient from different eye colorant samples.

In a next step, the composition of the coloring pigments and / or the additional pigments in dependence of the determined iris structure and the iris color distribution is selected. In this step, the different necessary coloring pigments and their compositions and / or the different necessary additional pigments and its composition is selected. In an embodiment, different available already manufactured composite material particles are selected or in another embodiment, the composite material particles are created with the matrix material, the selected coloring pigments and / or the selected additional pigments. The selection step is for example performed by a computing device, which automatically determines the required composition of the different components, based on user input.

In a next step, the selected composition of the different components of the eye colorant are merged for manufacturing the patient individual eye colorant. For example, the selected matrix material is merged with the selected coloring pigments and / or the additional pigments to form the composite material particles. Additionally, the composite material particles may be added to the selected carrier liquid comprising additional particles and / or additional coloring pigments to form the specific eye colorant. It is therefore possible to manufacture the unique, patient individual eye colorant based on the unique iris structure and iris color distribution of the eye of the patient or based on the target iris structure and target iris color distribution as desired by the patient.

In an optional further step, the composite material particles, the coloring pigments and / or the additional particles are sterilized. The particles are preferably sterilized using heat and / or gassing by means of EO (Ethylene Oxide). Sterilized particles are advantageously accepted tolerated within the cornea of the eye and reduce the risk of infections. The step of sterilization is for example performed prior or after the desired particle composition is selected and made.

In another embodiment, eye colorant is selected from a variety of available different eye colorants in dependence of the determined current iris structure and color distribution and / or the determined target iris structure and color distribution.

### BRIEF DESCRIPTION OF THE DRAWINGS

The herein described disclosure will be more fully understood from the detailed description given herein below and the accompanying drawings, which should not be considered limiting to the disclosure described in the appended claims. The drawings in which:
- **Figure 1**: shows a perspective view illustrating schematically an ophthalmological laser treatment system;
- **Figure 2**: shows an eye colorant according to a first embodiment;
- **Figure 3**: shows an eye colorant according to a second embodiment;
- **Figure 4**: shows an eye of a patient according to a first embodiment prior to treatment;
- **Figure 5**: shows the eye of the patient according to the first embodiment after treatment;
- **Figure 6**: shows an eye of a patient according to a second embodiment prior to treatment;
- **Figure 7**: shows an eye of the patient according to the second embodiment after treatment;
- **Figure** 8: shows a longitudinal section view of an eye of a patient according to a first embodiment;
- Figure 9: shows a longitudinal section view of an eye of a patient according to a second embodiment:
- Figure 10: shows a longitudinal section view of an eye of a patient according to a third embodiment;
- Figure 11: shows a block diagram illustrating schematically different steps of a method for manufacturing a patient individual eye colorant according to a first embodiment.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

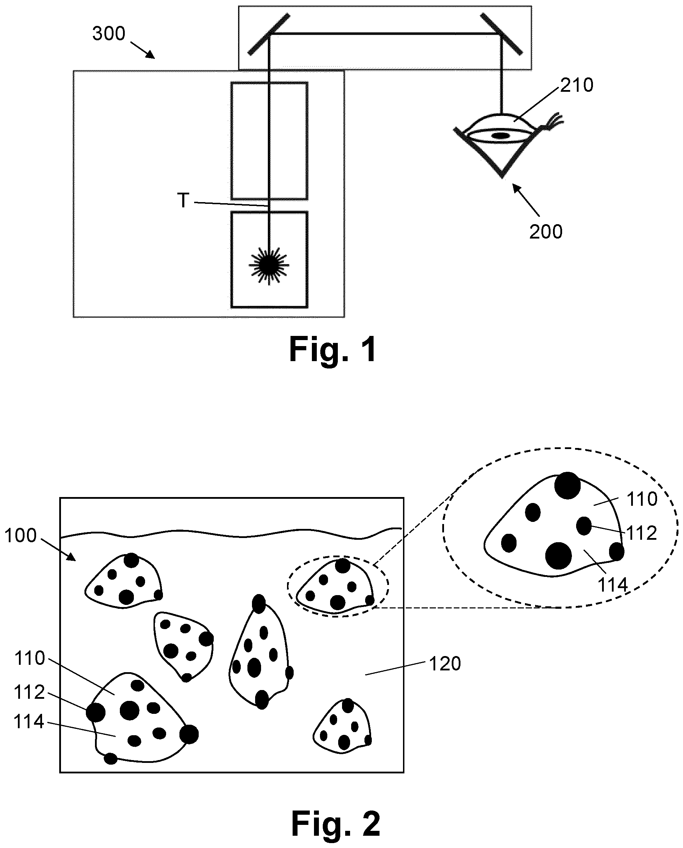

**Figure 1** schematically illustrates an ophthalmological laser treatment system 300 and a patient 200, in particular an eye 210 of the patient 200, which is treated with the ophthalmological laser treatment system 300. The ophthalmological laser treatment system 300 comprises a base station. The base station is for example a fixed or mobile apparatus. The ophthalmological laser treatment system 300 has a treatment laser source arranged in the base station, which generates a treatment laser beam T. The base station further includes, for example, a power supply and other auxiliary subsystems necessary for operation of the ophthalmological laser treatment system 300. The treatment laser source is configured, for example, to generate an infrared treatment laser beam T having a wavelength 780nm to 1100nm. In an embodiment, the treatment laser beam T is a pulsed laser beam. In an embodiment, the treatment laser source is configured to generate femtosecond laser pulses, which have pulse widths of typically from 10 fs to 1000 fs (1 fs = 10^-15 s). The ophthalmological laser treatment system 300 comprises a laser applicator or application head. The laser applicator is designed to guide the treatment laser beam T into or onto an eye 210 of a patient 200. The laser applicator can comprise focusing optics configured to focus the treatment laser beam T onto one or more treatment points inside or on the eye 210, in particular the cornea or the sclera for a pointwise tissue disruption or ablation. The ophthalmological laser treatment system 300 may comprise an ophthalmological patient interface. The laser applicator is preferably fixed onto the eye 210 by means of the ophthalmological patient interface, which is coupled to the eye for example using negative pressure.

The ophthalmological laser treatment system 300 optionally includes a user interface comprising, for example, one or more user input devices, such as a keyboard, and one or more output devices, such as a display (not shown in Figure 1). The display may also be an input device. The user interface is configured to receive user inputs from an eye treatment professional, in particular based on, or in response to, information displayed to the eye treatment professional using the one or more output devices.

**Figure 2** shows an eye colorant 100 according to a first embodiment. The eye colorant 100 comprises a plurality of composite material particles 110, which are distributed in a carrier liquid 120. The composite material particles 110 comprise matrix material 114 and coloring pigments 112. The coloring pigments 112 are at least partially embedded in the matrix material 114, thereby forming the composite material particles 110. The composite material particles 110 have all kind of different shapes, which are not limited to any specific shape. **Figure 2** shows the eye colorant 100 schematically in a container. The eye colorant 100 as shown in **Figure 2** is not at scale, it is illustrated at a larger scale. **Figure 2** further shows a detailed view of a single composite material particle 110.

In particular, the detailed view shows that the arbitrary arrangement of the coloring pigments 112 within the matrix material 110. The coloring pigments 112 determine at least partially the color of the eye colorant 100; in case the matrix material 114 and the carrier liquid 120 is fully transparent, the color of the eye colorant 100 is entirely determined by the coloring pigments 112. The coloring pigments 112 are for example merged or applied on the matrix material 114 in a liquid phase of the matrix material 114.

For example, the matrix material 114 is liquefied, e.g. melted and the coloring pigments 112 are added to the matrix material 114. In a next step, the matrix material 114 comprising the coloring pigments 112 is solidified, e.g. cured and the particles 110 are formed, for example by breaking the cured matrix material 114 into the desired size of the particles 110.

In another embodiment, the liquid matrix material 110 comprising the coloring pigments 112 is transformed into droplets having the desired size and cured afterwards for forming the composite material particles 110. The composite material particles 110 may be post-processed at a later stage, for example polished or coated.

The composite material particles 110 have an average equivalent diameter of below 25 micrometer, preferably of below 10 micrometer, more preferably of below 5 micrometer. Further, the composite material particles 110 have an average equivalent diameter of above 1 micrometer, preferably above 2 micrometer, more preferably above 4 micrometer. The average equivalent diameter is the average diameter of a number of particles of a sample of the composite material particles 110. The average equivalent diameter is for example the volume-equivalent sphere diameter or the surface-equivalent sphere diameter. As shown in **Figure 2****,** the coloring pigments 112 are of a single color within one single composite material particles 110 and within the plurality of the shown composite material particles 110. In another embodiment, one single composite material particles 110 may comprise a plurality of coloring pigments 112 of different colors. In a further embodiment, the eye colorant 100 comprises different composite material particles 110 with different coloring pigments 112.

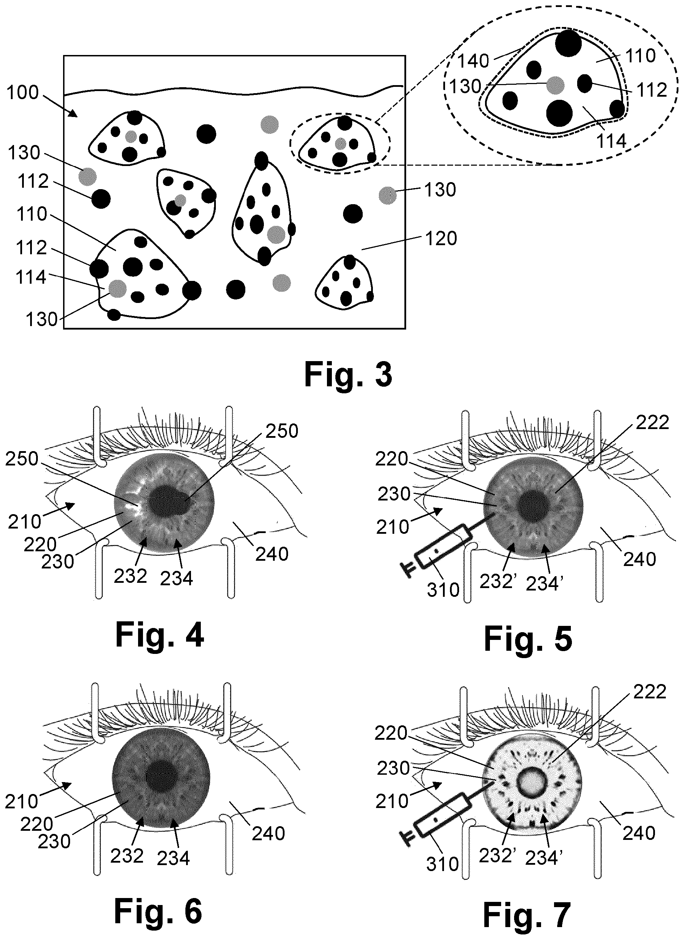

**Figure 3** shows a second embodiment of an eye colorant 100. This embodiment deviates from the first embodiment shown in **Figure 2** in that additional coloring pigments 112 are distributed in the carrier liquid 120 and, in that additional particles 130 are distributed in the carrier liquid 120. The coloring pigments 112 in the carrier liquid 120 additionally increase the possible variety of colors of the eye colorant 100. The additional particles 130 may comprise a light reflective or a light absorbing surface. The additional particles are for example carbon based. The additional particles 130 in the carrier liquid 120 and / or in the composite material particles 110 additionally increase the possible variety of colors of the eye colorant 100, in particular with respect to brightness, shininess or darkness.

The detailed view of **Figure 3****,** showing one single composite material particle 110, additionally shows a coating 140 around the single composite material particle 110. The coating 140, which, according to this embodiment entirely surrounds the composite material particles 110, is made of, for example, a biocompatible material or a medically or pharmaceutically active material. In a further embodiment, the additional particles 130 and / or the coloring pigments 112 distributed in the carrier liquid 120 may also be coated, in particular with the bio-compatible and / or medically or pharmaceutically active coating. The coating 140 prevents that the composite material particles 110 and / the additional particles 130 and / or the additional coloring pigments 112 get in direct contact with the cornea tissue of the patient 200, when the eye colorant 100 is inserted in the eye 210.

**Figure 4** shows an eye 210 of a patient 200 according to a first embodiment prior to treatment with the eye colorant 100. The eye 210 shows the sclera 240, the transparent cornea 220 and the natural iris 230 with its current iris structure 232 and its current iris color distribution 234. The iris 230 as shown in **Figure 4** comprises white spots 250, which affect the visual appearance of the iris 230. Other or additional spots on the iris 230 like melanoma are also conceivable. **Figure 4** further shows that the iris 230 comprises a defect 252, such that the iris 230 does not have a perfect ring shape. The iris defect 252 may result from a congenital disease such as coloboma. Such diseases or defects of the iris 230 or the cornea 220 of the eye affect the visual appearance of the eye 210 of the patient 200. The eye colorant 100 may be used to amend this visual appearance, as shown e.g. in **Figure 5****.**

**Figure 5** shows the eye 210 of the patient 200 according to the first embodiment after treatment with the eye colorant 100. **Figure 5** shows schematically a syringe 310, which has been used to insert the eye colorant 100 into a cornea pocket 222 of the eye 210. The eye colorant 100 is thereby inserted in front of the iris 230, with respect to the visual axis of the eye 210. The eye colorant 100 recreates the visual appearance of the desired color and structure of the iris 230 of the eye 210. The white spot 250 and the iris defect 252 is not removed, but they are covered by the eye colorant 100. The eye colorant 100 is according to this embodiment used to recreate a target iris structure 232' and a target iris color distribution 234'. In this embodiment, the target iris structure 232' and the target iris color distribution 234' corresponds to the current iris structure 232 and the current iris color distribution 234 without the white spot 250 and without the iris defect 252. The eye colorant 100 advantageously creates the desired visual appearance of the eye 210 of the patient 200.

**Figure 6** shows an eye 210 of a patient 200 according to a second embodiment prior to treatment with the eye colorant 100. The eye 210 of this second embodiment has a relative dark iris structure 232 and dark iris color distribution 234. In this embodiment, the eye 210 and in particular the iris 230 is not affected by a disease, but the patient 200 desires a change in the visual appearance of his iris 230. The eye colorant 100 may be used to change this visual appearance of the iris 230, as shown in **Figure 7****.**

**Figure 7** shows the eye 210 of the patient 200 according to the second embodiment after treatment with the eye colorant 100. The target iris structure 232' and the target iris color distribution 234', as selected by the patient 200, is brighter compared to the current iris structure 232 and the current iris color distribution 234 of the iris 230 of the patient 200. The patient 200 has, for example, selected the desired eye colorant 100, which is inserted into the cornea pocket 222 for the desired target iris structure 232' and the desired target iris color distribution 234'. The eye colorant 100 comprising the desired coloring pigments 112 and additional particles 130 advantageously creates the desired visual appearance of the iris 230 of the patient 210. The eye colorant 100 does not only enable to amend the visual appearance of the iris from dark to bright but also enables to amend the visual appearance of the iris with respect to its color structure and color distribution. It is for example possible to insert eye colorant 100 comprising mainly blue coloring pigments 112 to amend the visual appearance of the iris from any color into a blue color. Other colors are of course also conceivable.

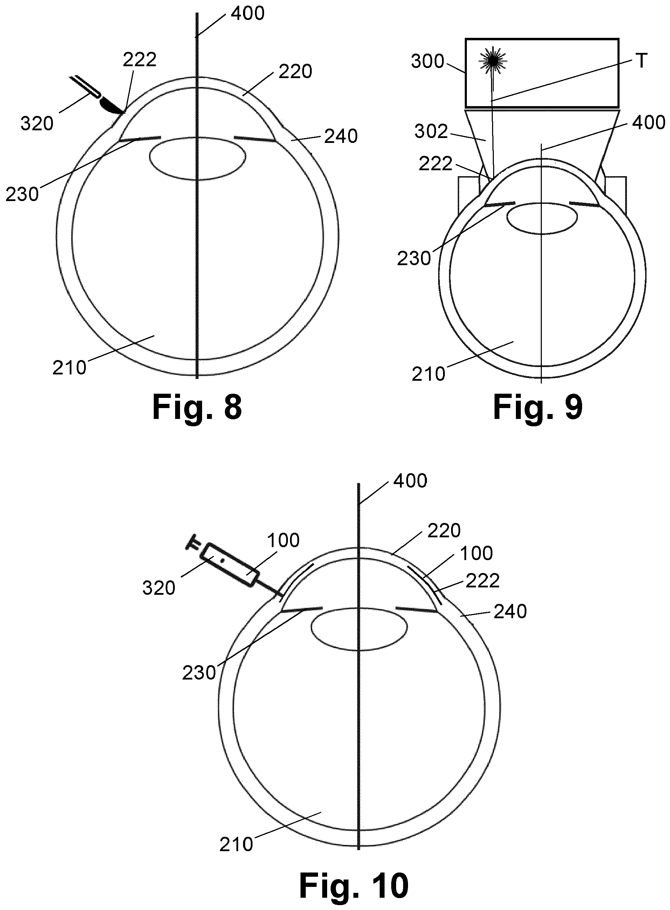

The **Figures 8** to **10** show different longitudinal section views of an eye 210 of a patient 200.

**Figure 8** shows a first longitudinal section view according to a first embodiment. The schematically shown eye 210 extends along its central axis 400. **Figure 8** further shows the different components of the eye 210. The eye 210 comprises the cornea 220, the iris 230 and the sclera 240. **Figure 8** further shows a scalpel 320, which is used to cut the pocket 222 into the portion of the cornea 220, the pocket 222 is configured to position the eye colorant 100 in the desired place of the cornea 220.

The pocket 222 may cover only a portion of the cornea 220, which is for example affected by a disease, or may have a ring shape for covering the entire cornea 220.

**Figure 9** shows a second longitudinal section view of an eye 210 according to a second embodiment. **Figure 9** shows the same components of the eye 210 as **Figure 8. Figure 9** shows an alternative possibility to cut the desired pocket 222 into the cornea 220 of the eye 210 of the patient 200. According to this embodiment, an ophthalmic laser treatment system 300, as for example described with reference to **Figure 1****,** is applied on the eye 210, which uses the treatment laser beam T to cut the pocket 222 into the cornea 220 of the eye 210. A combination of the ophthalmic laser treatment system 300 and a scalpel 320 as shown in **Figure 8** is also conceivable.

**Figure 10** shows a third longitudinal section view of an eye 210 of a patient 200 according to a third embodiment. **Figure 10** advantageously shows the pocket 222 for the eye colorant 100. The pocket 222 has the ring shape and follows the shape of the cornea 220 of the eye 210. According to this embodiment, the pocket 222 covers the entire iris 230 when viewed along the central axis 400. The eye colorant 100 inserted into the pocket 222 can thereby change the entire visual appearance of the entire iris 230, without affecting the iris 230 itself. **Figure 10** further shows schematically a syringe 320, which might be used to insert the eye colorant 100 into the pocket 222. An alternative for the syringe 320 is a spatula.

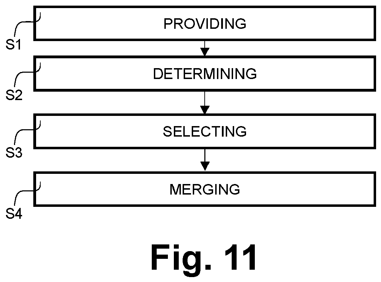

**Figure 11** shows a block diagram illustrating schematically different steps of a method for manufacturing a patient individual eye colorant 100. The patient individual eye colorant 100 may be used for coloring a portion of the cornea 220 of the eye 210 of the patient 200. The block diagram comprises the steps S1 to S4, additional or alternative steps are also conceivable.

In step S1, the different components of the eye colorant 100 are provided. The different components include at least the composite material particles 110 comprising the coloring pigments 112 and the matrix material 114. For example, different composite material particles 110, with different coloring pigments 112, are provided. The different components of the eye colorant 100 may further include carrier liquid 120, additional particles 130 and / or additional coloring pigments 112.

In step S2, the current iris structure 232 and / or a current iris color distribution 234 of the eye 210 of the patient 200 is determined. The current iris structure 232 and the current iris color distribution 234 is, for example, determined by using a digital image of the iris 230. The digital image is for example automatically processed by a specific device for determining the required information. For example, the iris 230 of the patient 200 comprises a white spot 250, which should be covered by a respective individual eye colorant 100. It is therefore necessary to determine the current iris structure 232 and / or a current iris color distribution 234 of a portion of the iris 230, which does not comprise the white spot. This portion of the iris 230 is for example the diametrically opposite portion of the iris 230 with respect to the white spot 250. In this embodiment, the eye colorant 100 recreates the original natural color of the iris 230 of the eye 210.

In an alternative embodiment, a target iris structure 232' and / or a target iris color distribution 234' is determined in step S2. The target iris structure 232' and / or the target iris color distribution 234' is for example selected by the patient 200 from a plurality of available predefined samples. The samples may cover all possibilities of natural eye colors and color distributions and may vary from light blue to dark brown. The patient 200 may select the desired target iris color, target color distribution 234' and target iris structure 232' based on an individual preference.

In step S3, the composition of the coloring pigments 112 and / or the additional pigments 130 and / or further components of the eye colorant 100 is selected in dependence of the determined iris structure 232, 232' and the iris color distribution 234, 234' is selected. For example, the specific device selects the composition of the different components of the eye colorant 100 such that the eye colorant has the determined properties, in particular the determined color distribution to achieve the desired visual appearance of the iris 230, when the eye colorant 100 is inserted in the pocket 222 of the cornea 220 of the eye 210.

In step S4, the selected composition of the different components of the eye colorant 100 is merged for manufacturing the patient individual eye colorant 100. For example, the specific device or a merging device combines the different components of the eye colorant 100 as selected in step S3 for manufacturing the individual eye colorant 100. In another embodiment, the different components are merged / combined by a professional manually based on the composition of the different components to create the desired individual eye colorant 100.

It should be noted that, in the description, the sequence of the steps has been presented in a specific order, one skilled in the art will understand, however, that the order of at least some of the steps could be altered or some steps could be skipped, without deviating from the scope of the disclosure.

### LIST OF REFERENCE SYMBOLS

- 100: eye colorant
- 110: composite material particles
- 112: coloring pigments
- 114: matrix material
- 120: carrier liquid
- 130: additional particles
- 140: coating
- 200: patient
- 210: eye
- 220: cornea
- 222: pocket
- 230: iris
- 232: current iris structure
- 232': target iris structure
- 234: current iris color distribution
- 234': target iris color distribution
- 240: sclera
- 250: white spot
- 252: iris defect
- 300: ophthalmic laser treatment system
- 302: patient interface
- 310: syringe or spatula
- 320: scalpel
- 400: central axis
- T: treatment laser beam
- S1: Providing
- S2: Determining
- S3: Selecting
- S4: Merging

## Claims

1. Eye colorant (100) for coloring a portion of a cornea (220) of an eye (210) of a patient (200), the eye colorant (100) comprising:
a. composite material particles (110) comprising:
i. coloring pigments (112) of at least one color and,
ii. a matrix material (114), which at least partially embeds the coloring pigments (112), thereby forming the composite material particles (110).

2. The eye colorant (100) according to claim 1, further comprising:
a. a carrier liquid (120), in which the composite material particles (110) are distributed.

3. The eye colorant (100) according to claim 2, further comprising at least one of:
a. coloring pigments (112), which are distributed in the carrier liquid (120), or
b. additional particles (130), which are distributed in the carrier liquid (120).

4. The eye colorant (100) according to one of the preceding claims, wherein the composite material particles (110) further comprises additional particles (130), which are also at least partially embedded within the matrix material (114).

5. The eye colorant (100) according to claims 3 or 4, wherein at least some of the additional particles (130) have a reflective surface and / or wherein at least some of the additional particles (130) have an absorbing surface.

6. The eye colorant (100) according to claim 5, wherein the additional particles (130) comprising the reflective surface are made of titanium dioxide.

7. The eye colorant (100) according to claim 5 or 6, wherein the additional particles (130) comprising the absorbing surface are made of a carbon based material.

8. The eye colorant (100) according to one of the preceding claims, wherein the composite material particles (110) are coated in at least one of: a coating (140) made of biocompatible material, a coating (140) made of a medical active material or a coating (140) made of a pharmaceutical active material.

9. The eye colorant (100) according to one of the preceding claims, wherein the matrix material (114) is made of at least one of: a biocompatible material, a medical active material, a pharmaceutical active material or transparent material.

10. The eye colorant (100) according to one of the preceding claims, wherein the matrix material (114) is made of a thermoplastic material, preferably made of a polymethyl methacrylate material, or wherein the matrix material (114) is made of a synthethic polymer material, preferably of a polyorganosiloxane material.

11. The eye colorant (100) according to one of the preceding claims, wherein the composite material particles (110) have an average equivalent diameter below 25 micrometer, preferably below 10 micrometer, more preferably below 5 micrometer.

12. The eye colorant (100) according to one of the preceding claims, wherein the coloring pigments (112) have a plurality of colors.

13. The eye colorant (100) according to one of the preceding claims, wherein the composition of the coloring pigments (112) is configured to be selected in dependence of:
a. a current iris structure (232) and / or a current iris color distribution (234) of the eye (210) of the patient (200) or,
b. a target iris structure (232') and / or a target iris color distribution (234') selected by the patient (200).

14. The eye colorant (100) according to one of the claims 3 to 13, wherein the composition of the additional particles (130) is configured to be selected in dependence of:
a. a current iris structure (232) and / or a current iris color distribution (234) of the eye (210) of the patient (200) or,
b. a target iris structure (232') and / or a target iris color distribution (234') selected by the patient (200).

15. The eye colorant (100) according to one of the preceding claims, wherein the surface of the composite material particles (110) has a hydrophobic property.

16. A method for manufacturing a patient individual eye colorant (100) for coloring a portion of a cornea (220) of an eye (210) of a patient (200), the method comprising the steps of:
a. providing (S1) the different components of an eye colorant (100) according to one of the claims 1 to 15;
b. determining (S2) a current iris structure (232) and / or a current iris color distribution (234) of the eye (210) of the patient (200) or, a target iris structure (232') and / or a target iris color distribution (234');
c. selecting (S3) the composition of at least one of: the coloring pigments (112) or the additional pigments (130) in dependence of the determined iris structure (232, 232') and the iris color distribution (234, 234');
d. merging (S4) the selected composition of the different components of the eye colorant (100) for manufacturing the patient individual eye colorant (100).
